# EUROPEAN PATENT SPECIFICATION

(11) **EP 1 694 249 B1**
(45) Date of publication and mention of the grant of the patent: **18.07.2007**
(21) Application number: 04812680.9
(22) Date of filing: 02.12.2004
(51) Int. Cl.: A61F 2/06

(54) **BILIARY STENT INTRODUCER SYSTEM**
BILIÄRSTENT-EINFÜHRSYSTEM
SYSTEME INTRODUCTEUR DE STENT BILIAIRE

(30) Priority: 04.12.2003 US 728589
(43) Date of publication of application: 30.08.2006
(73) Proprietor: WILSON-COOK MEDICAL INC., Winston-Salem North Carolina 27115-4191 (US)
(72) Inventor: CARTER, Matthew, P., Dobson, NC 27017 (US); CLARK, Victor, D., Jr., Winston-Salem, NC 27106 (US)
(74) Representative: Garratt, Peter Douglas
(86) International application number: PCT/US2004/040228
(87) International publication number: WO 2005/055882

(56) References cited:
- WO-A-02/30329
- WO-A-96/35470
- US-A- 4 994 066
- US-A- 5 571 135
- US-A1- 2002 161 341
- US-A1- 2003 149 444

## Description

### BACKGROUND

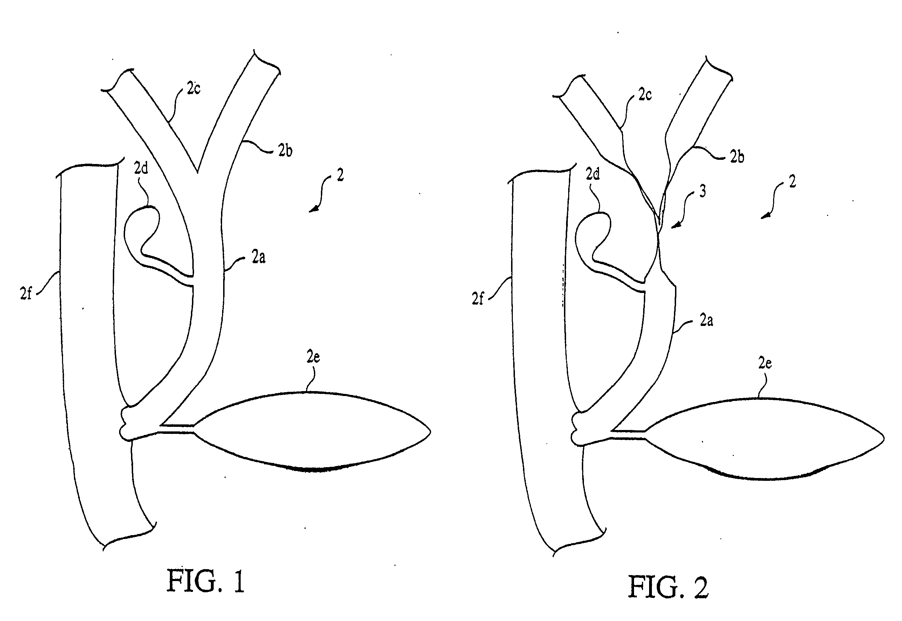

The invention generally relates to stent devices for maintaining the patency of the biliary tree, or common bile duct. Figure 1 is a partial, cross-sectional view of a biliary system 2 showing the common bile duct 2a, the left hepatic duct 2b, the right hepatic duct 2c, the gall bladder 2d, the pancreas 2e and the duodenum 2f.

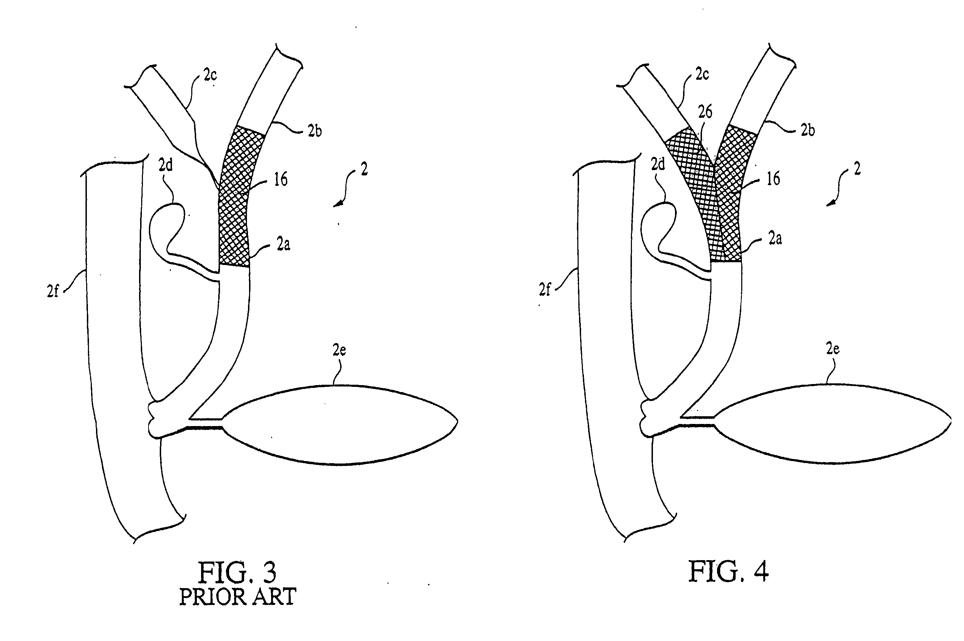

Strictures or occlusions that develop in the upper common bile duct and/or the left and right hepatic ducts can interfere with the proper drainage of those ducts. Figure 2 shows a partial, cross-sectional view of the biliary system 2 of Figure 1 showing strictures 3 within the common bile duct 2a, the left hepatic duct 2b and the right hepatic duct 2c. A successful method of treatment to reestablish proper drainage through the diseased ducts has been to open the ducts by placing prostheses, such as self-expanding biliary stents, within the restrictions. Because of the branched configuration of the duct anatomy it is often necessary to place two or more stents in an overlying or side-by-side configuration. However, currently available stent and introducer geometries are such that placement of a first stent may interfere with the placement of a second stent. Figure 3 illustrates the problem associated with the prior art method of placing stents in the common bile duct 2a and the left and right hepatic ducts 2b, 2c, *i.e.* the placement of a first stent 16 within the common bile duct 2a and the left hepatic duct 2b that obscures the access to the stricture in the right hepatic duct 2c.

Consequently, there is a need for a self-expanding stent delivery system which overcomes the problems associated with prior art delivery systems. Specifically, there is a need for a self-expanding stent delivery system which allows the physician to simultaneously place a first and second stent in the side branches and main lumen of a bifurcation.

### SUMMARY OF THE INVENTION

The invention relates to a stent delivery system according to claim 1. In one aspect of the invention, the stent delivery system includes a first introducer, a second introducer and an endoscope having a working channel. The first and second introducers are adapted to be disposed in an adjacent configuration within the working channel of the endoscope. The first introducer comprises a first outer catheter and a first inner shaft having a first stent retaining area located on a distal portion of the first inner shaft. The first inner shaft is located coaxially within the first outer catheter. The first outer catheter includes a first proximal outer catheter having a first proximal outer diameter and a first distal outer catheter having a first distal outer diameter. The distal end of the first proximal outer catheter is attached to a proximal end of the first distal outer catheter and the first proximal outer diameter is less than the first distal outer diameter.

The second introducer comprises a second outer catheter and a second inner shaft having a second stent retaining area located on a distal portion of the second inner shaft. The second inner shaft is located coaxially within the second outer catheter. The second outer catheter includes a second proximal outer catheter having a second proximal outer diameter and a second distal outer catheter having a second distal outer diameter. The distal end of the second proximal outer catheter is attached to a proximal end of the second distal outer catheter and the second proximal outer diameter is less than the second distal outer diameter.

In a second aspect of the invention, the stent delivery system comprises a first introducer, a second introducer and an endoscope having a working channel. The first and second introducers are adapted to be disposed in an adjacent configuration within the working channel of the endoscope. The first introducer comprises a first outer catheter, a first inner shaft located coaxially within the first outer catheter, a stent retaining area located on a distal portion of the first inner shaft, a first stent tip attached to a distal end of the first inner shaft, a first pusher band located proximate the first stent retaining area, a first stent mounted on the first stent retaining area such that the first stent proximal end abuts the first pusher band, and a first wire guide lumen extending proximally from the first introducer distal end through at least a portion of the first introducer. The first outer catheter includes a first proximal outer catheter having a first proximal outer diameter and a first distal outer catheter having a first distal outer diameter.

The second introducer comprises a second outer catheter, a second inner shaft located coaxially within the second outer catheter, a stent retaining area located on a distal portion of the second inner shaft, a second stent tip attached to a distal end of the second inner shaft, a second pusher band located proximate the second stent retaining area, a second stent mounted on the second stent retaining area such that the second stent proximal end abuts the second pusher band, and a second wire guide lumen extending proximally from the second introducer distal end through at least a portion of the second introducer. The second outer catheter includes a second proximal outer catheter having a second proximal outer diameter and a second distal outer catheter having a second distal outer diameter.

The stent delivery system of the present invention may be configured such that the sum of the first proximal outer diameter and the second distal outer diameter is less than the inner diameter of the working channel of the endoscope. Alternatively, the stent delivery system of the present invention may be configured such that the sum of the first proximal outer diameter, the second distal outer diameter and at least one of a first and second wire guide diameters is less than the inner diameter of the working channel of the endoscope. As another alternative, the stent delivery system may be configured such that the first proximal outer diameter is disposed adjacent to the second distal outer diameter while inside the working channel of the endoscope.

In an aspect not being part of the invention, the method of placing a first stent within a first branch lumen and a main lumen of a bifurcation and placing a second stent within a second branch lumen and the main lumen of the bifurcation comprises the steps of: providing an endoscope having a first introducer with the first stent retained on a first distal portion and a second introducer having the second stent retained on a second distal portion disposed in an adjacent configuration within a working channel of the endoscope; placing a first wire guide into the main lumen and the first branch lumen of the bifurcation and placing a second wire guide into the second branch lumen and the second branch lumen of the bifurcation, and advancing the first introducer over the first wire guide into the main lumen and the first branch lumen of the bifurcation and advancing the second introducer over the second wire guide into the main lumen and second branch lumen of the bifurcation such that the first and second introducers are simultaneously positioned within the main lumen and the first and second branch lumens of the bifurcation. The method may further comprise the step of: deploying, sequentially or simultaneously, the first stent within the first branch lumen and the main lumen of the bifurcation.

A method of placing first and second stents in first and second branch lumens and a main lumen of a bifurcation is described, comprising the steps of: positioning the first stent within the first branch and the main lumen of the bifurcation such that a distal portion of the first stent extends at least partially within the first branch of the bifurcation and a proximal portion of the first stent extends at least partially within the main lumen of the bifurcation; positioning the second stent within the second branch and the main lumen of the bifurcation such that a distal portion of the second stent extends at least partially within the second branch of the bifurcation and a proximal portion of the second stent extends at least partially within the main lumen of the bifurcation; and deploying the first and second stents within the bifurcation.

### BRIEF DESCRIPTION OF THE DRAWINGS

Figure 1 is a partial, cross-sectional view of a biliary system showing the common bile duct, the left hepatic duct, the right hepatic duct, the gall bladder, the pancreas and the duodenum.

Figure 2 is a partial, cross-sectional view of the biliary system of Figure 1 showing strictures within the common bile duct, the left hepatic duct and the right hepatic duct.

Figure 3 is a partial, cross-sectional view of the biliary system of Figure 2 illustrating a stent that has been placed in the common bile duct and the left hepatic duct.

Figure 4 is a partial, cross-sectional view of the biliary system of Figure 2 illustrating the placement of first and second stents in the left and right hepatic ducts, respectively, and the common bile duct.

Figure 5 is a cross-sectional view of a preferred embodiment of the first and second introducers of the stent delivery system of the present invention.

Figure 6 is a partial, cross-sectional view of a distal portion of the first introducer of Figure 5.

Figure 6A is a partial, cross-sectional view of a distal portion of the second introducer of Figure 5.

Figure 7 is a partial, cross-sectional view of an alternate preferred embodiment of the distal portion of the first introducer of Figure 5.

Figure 7A is a partial, cross-sectional view of an alternate preferred embodiment of the distal portion of the first introducer of Figure 5.

Figure 8 is a partial, cross-sectional view of the distal portion of the first introducer of Figure 5 showing the wire guide and wire guide lumen.

Figure 8A is a partial, cross-sectional view of the distal portion of the second introducer of Figure 5 showing the wire guide and wire guide lumen.

Figure 9 is a partial, cross-sectional view of the distal portion of the first introducer of Figure 5 showing an alternate embodiment of the wire guide and the wire guide lumen.

Figure 9A is a partial, cross-sectional view of the distal portion of the second introducer of Figure 5 showing an alternate embodiment of the wire guide and the wire guide lumen.

Figure 10 is a partial, cross-sectional view of the distal portion of the first introducer of Figure 5 showing an alternate embodiment of the wire guide and the wire guide lumen.

Figure 10A is a partial, cross-sectional view of the distal portion of the second introducer of Figure 5 showing an alternate embodiment of the wire guide and the wire guide lumen.

Figure 11 is a cross-sectional, end view of the stent delivery system of the present invention showing the first and second introducers within the working channel of an endoscope.

Figure 12 is a partial, cross-sectional, side-view of a preferred embodiment of the stent delivery system of the present invention showing the first and second introducers within the working channel of an endoscope.

Figure 13 is a partial, cross-sectional view of the biliary system of Figure 2 illustrating the stent delivery system of the present invention and the placement of first and second introducers in the left and right hepatic ducts, respectively, and the common bile duct according to a preferred method of the present invention.

### DETAILED DESCRIPTION OF THE PREFERRED EMBODIMENTS

Referring now to the Figures wherein like numerals indicate the same element throughout the views, there is shown in Figures 1-2 and 4 a bifurcation comprising a main lumen, a first branch lumen and a second branch lumen. In particular, these figures illustrate a bifurcation in the biliary system, wherein the main lumen comprises the common bile duct 2a and the first and second branch lumens comprise the left and right hepatic ducts 2b, 2c respectively. Figure 1 shows a normal, or healthy, biliary system with no strictures. Figure 2 shows the biliary system with strictures 3 residing in the main lumen and in both branch lumens of the bifurcation. Figure 4 shows a pair of stents placed in the left and right hepatic ducts 2b, 2c, respectively, and the common bile duct 2a according to a preferred method of the present invention.

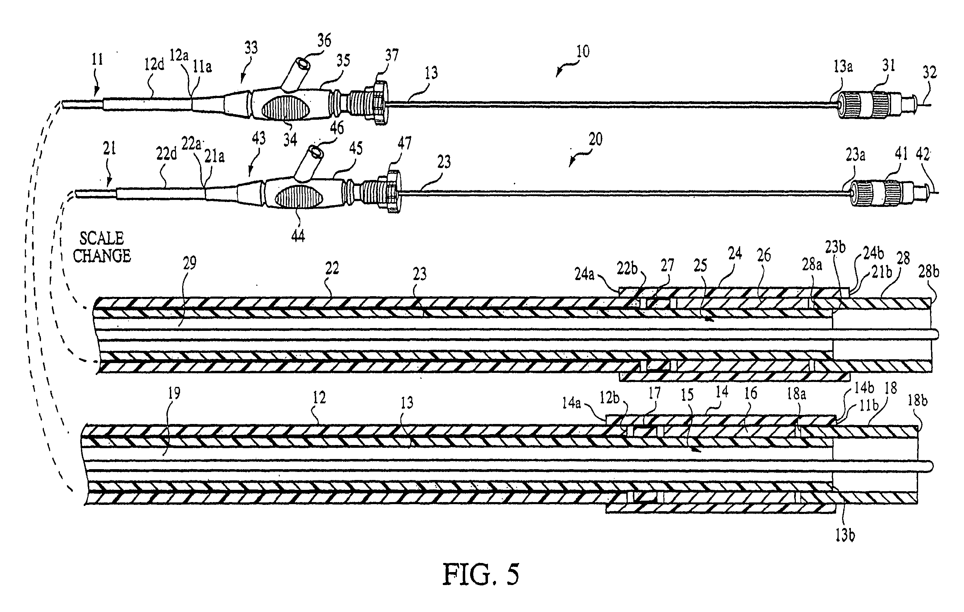

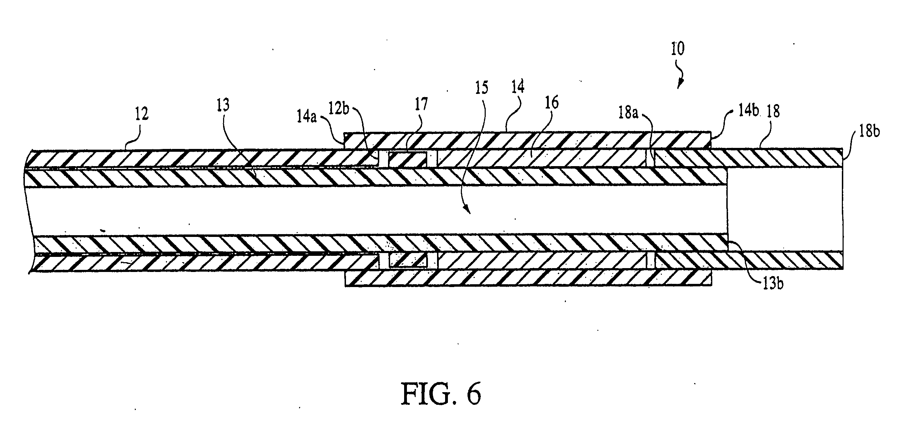

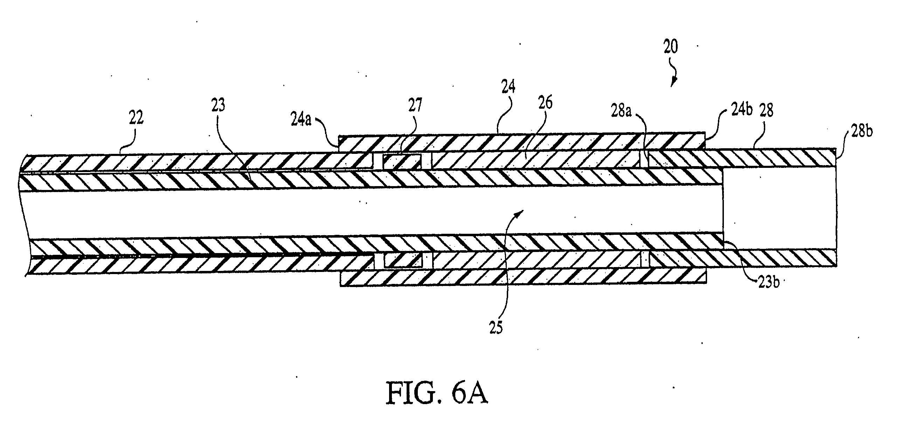

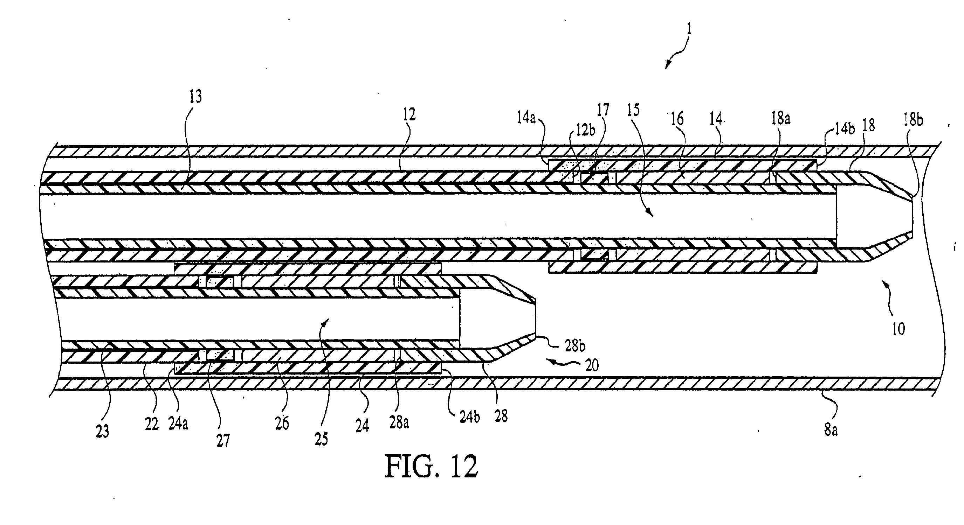

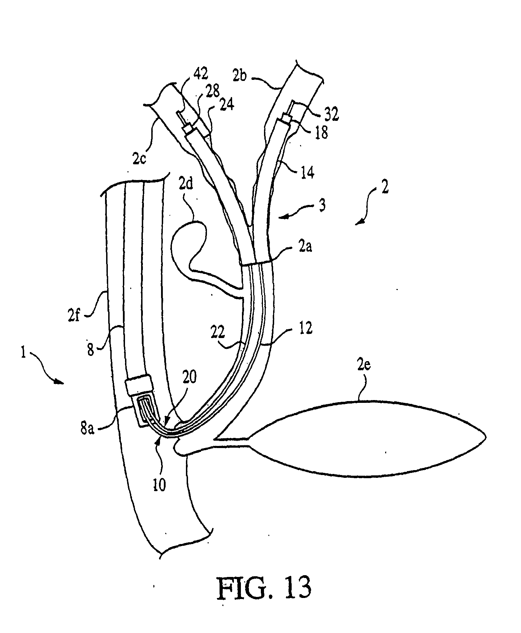

Referring now to Figures 12 and 13, a stent delivery system 1 made in accordance with the present invention is shown. Stent delivery system 1 comprises a first introducer 10 and a second introducer 20 disposed within the working channel 8a of an endoscope 8. Both first and second introducers 10, 20 are similar with respect to the views shown in Figures 5-13. Therefore, reference will be made to both first and second introducers 10, 20 in the description below.

As shown in Figure 5, introducer 10, 20 has a proximal end and a distal end and comprises inner and outer coaxial tubes. The outer coaxial tube is called an outer catheter, or sheath, 11,21. The inner coaxial tube is called a shaft 13, 23.

Shaft 13, 23 has a proximal end 13a, 23a, a distal end 13b, 23b and a stent retaining area 15, 25. Optionally, shaft 13, 23 may include a pusher band 17, 27 attached to the stent retaining area 15, 25, a distal tip 18, 28 attached to the shaft distal end 13b, 23b and a wire guide lumen 19, 29. Shaft 13, 23 can be made from any suitable material known in the art including, but not limited to, polyethylene ether ketone (PEEK), polytetrafluoroethylene (PTFE), polyamide, polyurethane, polyethylene and nylon, including multi-layer or single layer structures and may also include reinforcement wires, braid wires, coils and or filaments. Preferably, shaft 13, 23 comprises a proximal portion made of a relatively rigid material such as stainless steel or any other suitable material known in the art.

Stent retaining area 15, 25 is preferably located on a distal portion of the shaft 13, 23. The stent retaining area 15, 25 retains a stent 16, 26 to be deployed in the bifurcation. Preferably, stent 16, 26 is a self-expanding stent.

Pusher band 17, 27 helps to prevent the stent from proximally migrating as the outer catheter 11, 21 is withdrawn proximally to deploy the stent. The pusher band 17, 27 is located proximal to the stent 16, 26 such that the proximal end of the stent 16, 26 abuts the pusher band 17, 27 as shown in Figures 5-10A and 12.

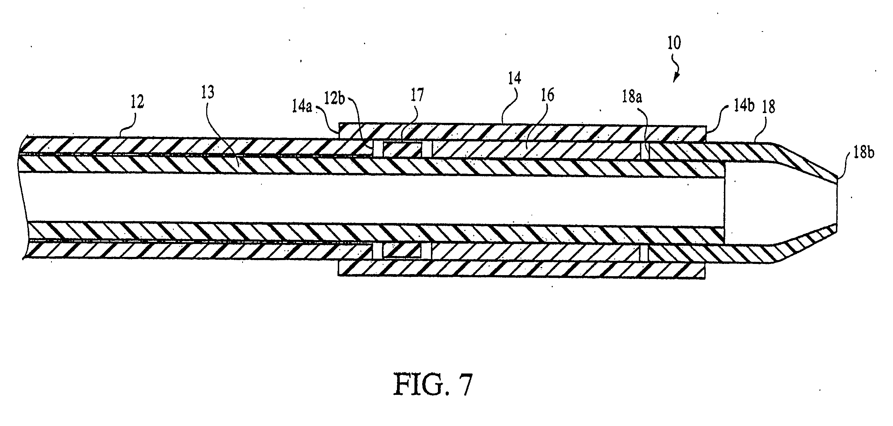

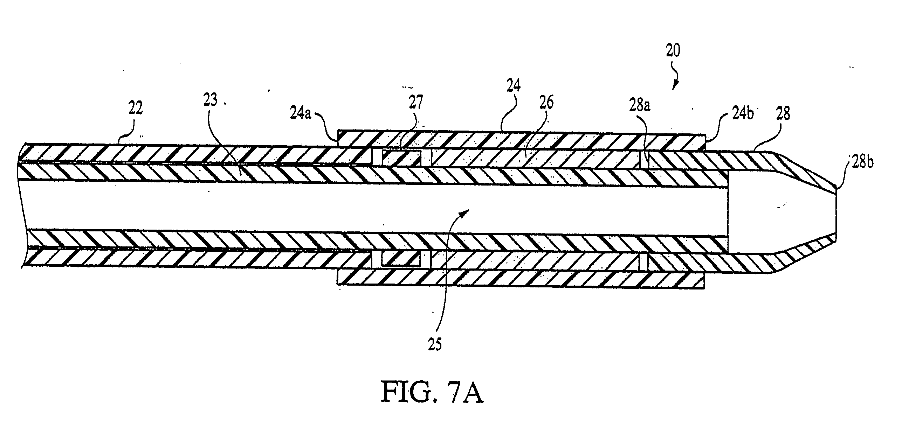

Distal tip 18, 28 helps prevent fluids from entering the outer catheter 11, 22 as the introducer 10, 20 is navigated through the body lumens. As shown in Figures 5-10A and 12, distal tip 18,28 has a proximal end 18a, 28a and a distal end 18b, 28b. The distal tip proximal end 18a, 28a has a diameter that is less than the diameter of the distal outer catheter distal end 14b, 24b and is received therein. Preferably, the distal tip 18, 28 tapers to a smaller diameter towards its distal end 18b, 28b as shown in Figures 7, 7A and 12. Distal tip 18, 28 can be made from any suitable material known in the art including, but not limited to, PEEK, PTFE, polyamide, polyurethane, polyethylene and nylon, including multi-layer or single layer structures.

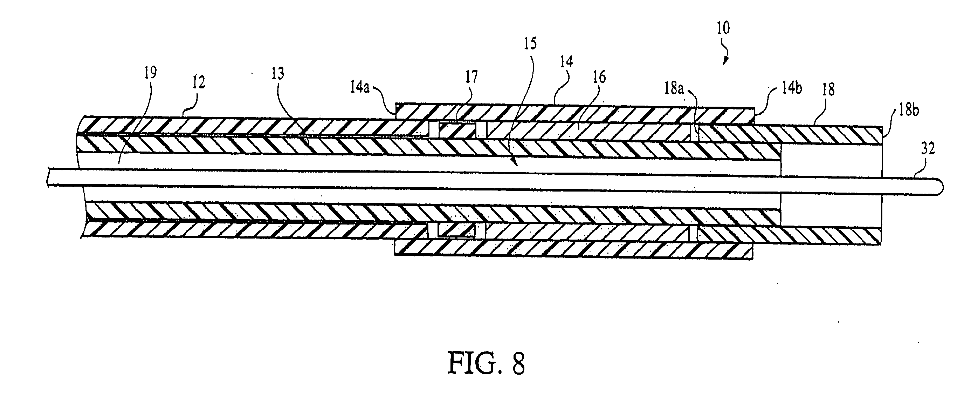

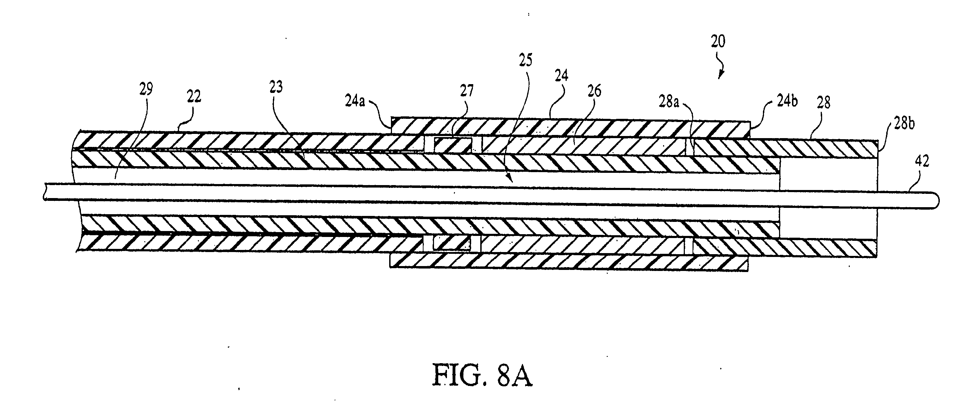

In the embodiment shown in Figures 5, 8 and 8A, wire guide lumen 19, 29 extends through the shaft 13, 23 from the shaft distal end 13b, 23b to the shaft proximal end 13a, 23a. In this embodiment, the shaft proximal end 13a, 23a preferably includes a luer-lock fitting 31, 41 for releaseably fixing a wire guide 32, 42 relative to shaft 13, 23 as shown in Figure 5. In the embodiments shown in Figures 5, 8 and 8A, the stent delivery system 1 of the present invention includes an over-the-wire type wire guide. Such wire guides are known in the art.

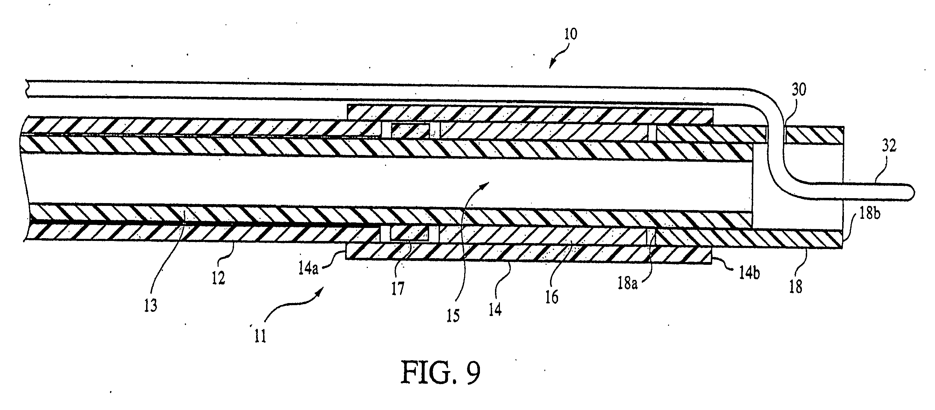

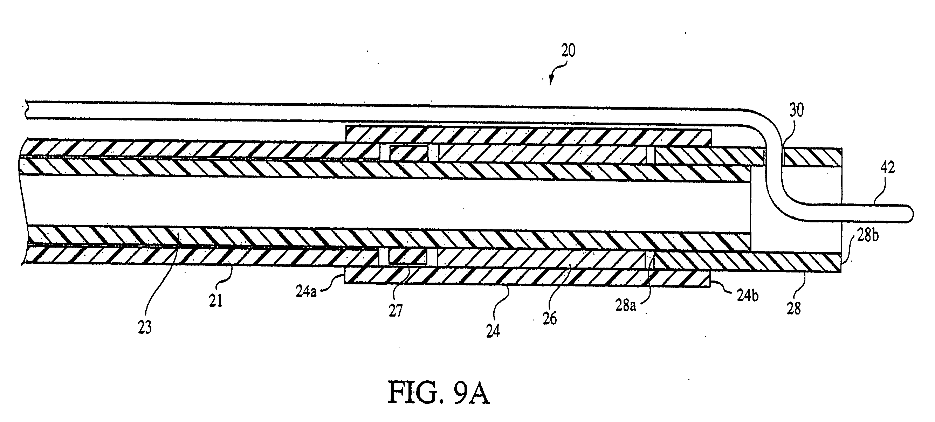

Alternatively, the wire guide lumen 19, 29 may extend through the shaft 13, 23 from the shaft distal end 13b, 23b to the shaft proximal end 13a, 23a but the wire guide 32, 42 exits through an aperture positioned along the length of the introducer 10, 20. For example, as shown in Figures 9 and 9A, the wire guide lumen 19, 29 extends through the length of the shaft 13, 23 but the wire guide 32, 42 extends through a portion of the distal tip 18, 28 and exits through an aperture 30, 40 positioned along the length of the distal tip 18, 28. In this embodiment, the wire guide 32, 42 extends through the distal tip 18, 28 and exits the introducer 10, 20 without passing through stent 16, 26. For example, wire guide 32, 42 may extend proximally through distal tip 18, 28 for a distance of about 1 cm.

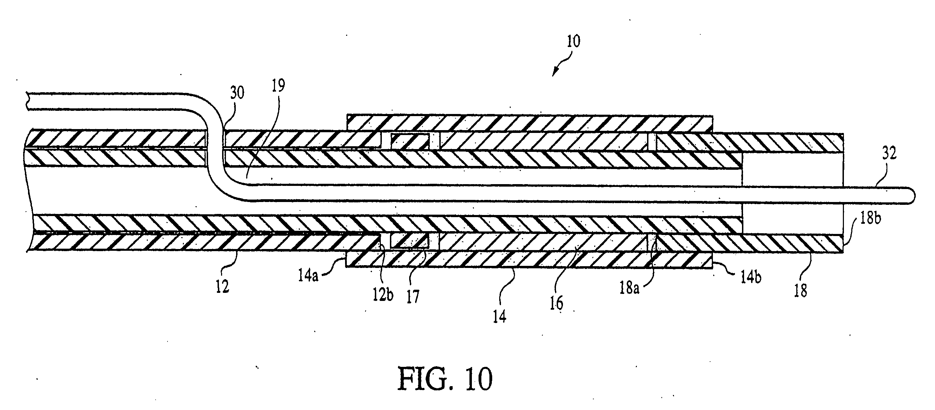

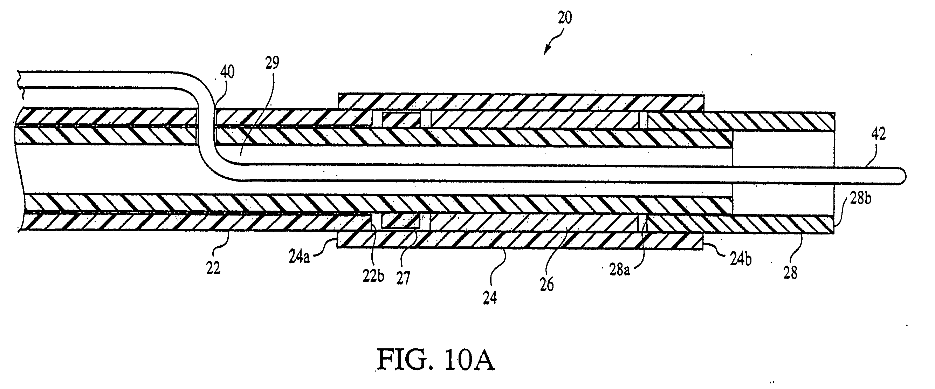

In the alternate embodiment shown in Figures 10 and 10A, the wire guide lumen 19, 29 extends through the length of the shaft 13, 23 but the wire guide 32, 42 extends through a portion of the shaft 13, 23 and exits through an aperture 30, 40 positioned along the length of outer catheter 11, 21. In this embodiment, wire guide 32, 42 extends through the distal tip 18, 28, through a portion of the shaft 13, 23 and passes through stent 16, 26 before exiting introducer 10, 20. For example, wire guide 32, 42 may extend through the distal tip 18, 28 and through the stent retaining area 15, 25 for a distance of about 20 cm.

In yet other alternative embodiments, the wire guide lumen 19,29 may extend through a portion of shaft 13, 23 and may exit through an aperture 30, 40 positioned along the length of the introducer 10, 20. Any number of apertures 30, 40 positioned at any location along the length of the introducer 10, 20 is contemplated. In addition, the wire guide lumen 19, 29 may also comprise a channel or split.

Aperture 30, 40 provides the stent delivery system of the present invention with rapid-exchange capabilities. In particular, by extending the wire guide 32, 42 through only a distal portion of the wire guide lumen 19, 29, the delivery system can be removed from a wire guide 32, 42 having a length substantially shorter than the length necessary if the wire guide 32, 42 were extended through the entire length of the wire guide lumen 19, 29.

Referring to Figure 5, outer catheter, or sheath, 11, 21 has a proximal end 11a, 21a and a distal end 11b, 21b. The outer catheter, or sheath, 11,21 further comprises a proximal outer catheter 12, 22 having proximal and distal ends, 12a, 22a and 12b, 22b respectively, and a distal outer catheter 14, 24 having proximal and distal ends, 14a, 24a and 14b, 24b respectively. The distal end 12b, 22b of the proximal outer catheter 12, 22 is attached to the proximal end 14a, 24a of the distal outer catheter 14, 24 to form outer catheter 11, 22.

In one embodiment, the proximal outer catheter 12, 22 and the distal outer catheter 14, 24 comprise separate catheters, or sheaths as shown in Figures 5-10A and 12. The distal end 12b, 22b of proximal outer catheter 12, 22 can be attached to the proximal end 14a, 24a of distal outer catheter 14, 24 by any method known in the art including, but not limited to, heat fusing, adhesive bonding, chemical bonding or mechanical fitting. In an alternate embodiment (not shown), the proximal outer catheter 12, 22 and the distal outer catheter 14, 24 comprise portions of a single catheter or sheath.

Proximal outer catheter 12, 22 further comprises a proximal outer diameter and the distal outer catheter 14, 24 further comprises a distal outer diameter. In one embodiment the sum of the first outer catheter diameter and the second outer catheter diameter is less than the inner diameter of the working channel 8a of the endoscope 8.

Referring to the embodiment shown in Figure 12, a pair of introducers 10, 20 are disposed next to each other in the working channel 8A of an endoscope 8. The introducers 10, 20 are adapted so that the overall diameter of the pair of introducers 10, 20 is minimized. For this reason, the diameter of the portion of the introducer 10, 20 that retains the stent 16, 26 is sized to accommodate the stent 16, 26 in its compressed or loaded configuration and the portion proximal to the stent retaining area 15, 25 of the introducer 10, 20 is minimized. That is, the proximal outer diameter is less than the distal outer diameter. In this embodiment, the overall diameter of the pair of introducers 10, 20 is minimized by juxtaposing the stent retaining area of a first introducer 10 with the portion proximal to the stent retaining area of a second introducer 20. As can be seen in Figure 12, the sum of the first proximal outer diameter and the second distal outer diameter is less than the inner diameter of the working channel 8a of the endoscope 8. Alternatively, the sum of the outer diameter of the first distal outer catheter 14 and the outer diameter of the second proximal outer catheter 22 is less than the inner diameter of the working channel 8a of the endoscope 8.

In yet another alternate embodiment of the stent delivery system 1 of the present invention, the pair of introducers 10, 20 may be sized to also accommodate a wire guide 32, 42 within the working channel 8A of the endoscope 8. For this embodiment, the sum of the outer diameter of the first proximal outer catheter 12, the outer diameter of the second distal outer catheter 24 and the diameter of at least one of the first and second wire guides 32, 42 is less than the inner diameter of the working channel 8a of the endoscope 8.

Preferably, the outer diameter of proximal outer catheter 12, 22 is about 5 to about 6 French and the outer diameter of the distal outer catheter 14, 24 is about 6 to about 7 French to place a stent 16, 26 having a compressed diameter of about 1,75 mm (0.069 inches) to about 2 mm (0.077 inches). More preferably, the proximal outer catheter 12, 22 is about 5.5 French and the outer diameter of the distal outer catheter 14, 24 is about 6.5 French. These sizes are provided for illustrative purposes only and are not intended to be construed as a limitation of the present invention. As one of ordinary skill in the art would appreciate, the size of the introducer required to place a stent is related to the size of the stent to be placed, and more particularly, to the size of the compressed configuration of the stent. Thus, introducers 10, 20 having distal outer catheter diameters less than about 6 French used to place stents having compressed configurations less than about 1,75 mm (0.069 inches) that may become available in the future are contemplated as being within the scope of the claims of the invention.

Returning to Figure 5, introducer 10, 20 may further comprise a handle 33, 43 attached to proximal outer catheter 12, 22 proximal end 12a, 22a. Handle 33, 43 may optionally comprise a gripping member 34, 44 a connector 35, 45 having an injection port 36, 46 and/or a releasable locking mechanism 37, 47. Injection port 36, 46 is used to irrigate the shaft 13, 23 and stent 16, 26 with sterile water prior to use. Releaseable locking mechanism 37, 47 releasably affixes shaft 13, 23 to outer catheter 11,21.

Preferably, proximal outer catheter 12, 22 further comprises a support portion 12d, 22d adjacent to the handle 33, 43 as shown in Figure 5. Preferably, the support portion 12d, 22d of the proximal outer catheter 12, 22 is not positioned within the working channel 8a of the endoscope 8. Support portion provides additional column strength to the introducers 10, 20 as the introducers 10, 20 are manipulated through the lumens of a patient. Support portion 12d, 22d may comprise a separate support catheter having an outer diameter of about 7 to about 9 French. Preferably, the outer diameter of support portion 12d, 22d is about 7 French.

Outer catheter, or sheath, 11, 21 can be made from any suitable material known in the art including, but not limited to, PTFE, polyamide, polyurethane, polyethylene and nylon including multi-layer or single layer structures and may also include reinforcement wires, braid wires, coils and or filaments. Preferably, at least the distal portion of outer catheter 11, 21 is made of any relatively clear material so that the stent 16, 26 mounted on the stent retaining area 15, 25 of the shaft 13, 23 can be viewed.

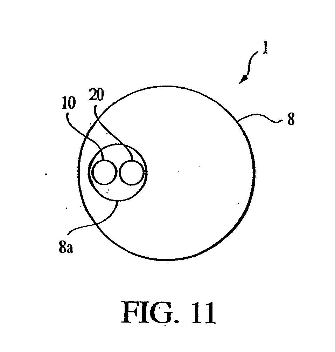

The stent delivery system 1 of the present invention is used to place first and second stents 16, 26 into a bifurcation having strictures 3 in the main lumen 2a and the first and second branch lumens 2b, 2c as follows. A distal end of a first wire guide 32 is advanced through the main lumen and into the first branch lumen of the bifurcation and a distal end of a second wire guide 42 is placed through the main lumen and into the second branch lumen of the bifurcation. The proximal ends of the first and second wire guides 32, 42 are passed through the accessory channel 8a of an endoscope 8. The first and second introducers 10, 20 are inserted into the working channel 8a of the endoscope 8. First and second introducers 10, 20 may be positioned side-by-side within the working channel 8a of the endoscope 8 as shown in the embodiment of Figure 11. Alternatively, the first and second introducers 10, 20 may be positioned so that the proximal outer catheter 12 of the first introducer 10 is positioned adjacent the distal outer catheter 24 of the second introducer 20 as shown in Figure 12. The first and second introducers 10, 20 are advanced over the first and second wire guides, 32, 42 respectively, such that the first introducer 10 is positioned within the main lumen 2a and the first branch lumen 2b of the bifurcation and the second introducer 20 is positioned within the main lumen 2a and the second branch lumen 2c of the bifurcation as shown in Figure 13. The first introducer 10 and the second introducer 20 may be positioned sequentially or simultaneously. The first and second introducers 10, 20 are positioned such that the first and second stents 16, 26, retained within the stent retaining area 15, 25 are at least partially aligned within the first and second branch lumens 2b, 2c, respectively, and the main lumen 2a of the bifurcation. Once aligned, the first and second outer catheters 11, 21 are withdrawn proximally to deploy the first and second stents 16, 26 within the first and second branch lumens 2b, 2c, respectively, and the main lumen 2a of the bifurcation. First and second stents 16, 26 may be deployed sequentially or simultaneously.

The above Figures and disclosure are intended to be illustrative and not exhaustive. This description will suggest many variations and alternatives to one of ordinary skill in the art. All such variations and alternatives are intended to be encompassed within the scope of the attached claims. Those familiar with the art may recognize other equivalents to the specific embodiments described herein which equivalents are also intended to be encompassed by the attached claims.

## Claims

1. A stent delivery system comprising:
a) a first introducer having proximal and distal ends comprising:
i) a first outer catheter having proximal and distal ends, the first outer catheter comprising:
A) a first proximal outer catheter having proximal and distal ends and a first proximal outer diameter, and
B) a first distal outer catheter having proximal and distal ends and a first distal outer diameter,
wherein the first proximal outer catheter distal end is connected to the first distal outer catheter proximal end;
wherein the first proximal outer diameter is less than the first distal outer diameter;
ii) a first inner shaft located coaxially within said first outer catheter, the first inner shaft having proximal and distal ends, a first stent retaining area located on a distal portion of the first inner shaft; and
b) a second introducer having proximal and distal ends comprising:
i) a second outer catheter having proximal and distal ends, the second outer catheter comprising:
A) a second proximal catheter having proximal and distal ends and second proximal outer diameter; and
B) a second distal catheter having proximal and distal ends and a second distal outer diameter,
wherein the second proximal outer diameter is less than the second distal outer diameter; and
wherein the second proximal catheter distal end is connected to the second distal catheter proximal end;
ii) a second inner shaft located coaxially within said second outer catheter, the second shaft having proximal and distal ends, a second stent retaining area located on a distal portion of the second inner shaft;
wherein said first and second introducers are adapted to be disposed in an adjacent configuration having an overall diameter; and
wherein the overall diameter is less than the sum of the first distal outer diameter and the second distal outer diameter.

2. The stent delivery system of claim 1 wherein the first introducer further comprises a first stent having proximal and distal ends mounted on the first stent retaining area of the first inner shaft.

3. The stent delivery system of claim 2, wherein the first introducer further comprises a first pusher band attached to the first inner shaft.

4. The stent delivery system of claim 3 wherein the proximal end of the first stent abuts the first pusher band.

5. The stent delivery system of any one of the preceding claims, wherein the first introducer further comprises a first wire guide lumen.

6. The stent delivery system of claim 5, wherein the first wire guide lumen extends through at least a portion of the first introducer.

7. The stent delivery system of claim 5, wherein the first wire guide lumen extends through at least a portion of the first inner shaft.

8. The stent delivery system of claim 5, wherein the first wire guide lumen extends proximally from the first introducer distal end for a distance of up to about 20 cm, or up to about 1cm.

9. The stent delivery system of any one of the preceding claims, wherein the first introducer further comprises a first stent tip attached to the distal end of the first inner shaft, the first steal tip being preferably tapered.

10. The stent delivery system of any one of the preceding claims, wherein the first proximal outer catheter and the first distal outer catheter comprise two separate catheters.

11. The stent delivery system of any one of the preceding claims, wherein the sum of the first proximal outer diameter and the second distal outer diameter is less than the overall diameter.

12. The stent delivery system of any one of the preceding claims wherein the sum of the first proximal outer diameter and the second distal outer diameter and at least one of a first and second wire guide diameters is less than the overall diameter.

13. The stent delivery system of any one of the preceding claims, wherein the first proximal outer catheter is disposed adjacent to the second distal outer catheter.

14. The stent delivery system of any one of the preceding claims, wherein the first proximal outer diameter is disposed adjacent to the second distal outer diameter.

15. The stent delivery system of any one of the preceding claims wherein the overall diameter is about 3.0 to about 4.5 mm.

16. The stent delivery system of any one of the preceding claims wherein said the first proximal outer diameter is about 5.0 to about 6.0 French.

17. The stent delivery system of any one of the preceding claims wherein the first distal outer diameter is about 6.0 to about 7.0 French.

18. The stent delivery system of any one of the preceding claims, further comprising:, a second stent retaining arca located on a distal portion of the second inner shaft, a second pusher band located proximate the second stent retaining area;
with a second stent tip preferably attached to the distal end of the second inner shaft there being
a second stent having proximal and distal ends preferably mounted on the second stent retaining area of the second inner shaft such that the second stent proximal end abuts the second pusher band;

19. The stent delivery system of any one of the preceding claims, further comprising a second wire guide lumen extending proximally from the second introducer distal end through at least a portion of the second introducer.

## Patentansprüche

1. Stent-Abgabesystem umfassend:
a) eine erste Einführvorrichtung mit proximalem und distalem Ende, umfassend:
i) einen ersten äußeren Katheter mit proximalem und distalem Ende, wobei der erste äußere Katheter umfasst:
A) einen ersten äußeren Katheter mit proximalem und distalem Ende und einem ersten Außendurchmesser und
B) einen ersten distalen äußeren Katheter mit proximalem und distalem Ende und einem ersten distalen Außendurchmesser,
wobei das distale Ende des ersten proximalen äußeren Katheters mit dem proximalen Ende des ersten distalen äußeren Katheters verbunden ist,
wobei der erste proximale Außendurchmesser geringer ist als der erste distale Außendurchmesser;
ii) ein erster innerer Schaft, welcher coaxial innerhalb des ersten äußeren Katheters positioniert ist, wobei der erste innere Schaft ein proximales und ein distales Ende aufweist und einen ersten Stent aufnehmenden Bereich, welcher an einem distalen Abschnitt des ersten inneren Schafts positioniert ist und
b) eine zweite Einführvorrichtung mit einem proximalen und einem distalen Ende, umfassend:
i) einen zweiten äußeren Katheter mit einem proximalen und einem distalen Ende, wobei der zweite äußere Katheter umfasst:
A) einen zweiten proximalen Katheter mit einem proximalen und einem distalen Ende und einem zweiten proximalen Außendurchmesser und
B) einen zweiten distalen Katheter mit einem proximalen und einem distalen Ende und einem zweiten distalen Außendurchmesser,
wobei der zweite proximale Außendurchmesser geringer ist als der zweite distale Außendurchmesser und
wobei das distale Ende des zweiten proximalen Katheters mit dem proximalen Ende des zweiten distalen Katheters verbunden ist;
ii) ein zweiter innerer Schaft, welcher coaxial innerhalb des zweiten äußeren Katheters positioniert ist, wobei der zweite Schaft ein proximales und ein distales Ende aufweist und einen zweiten Stent aufnehmenden Bereich, der an einem distalen Abschnitt des zweiten inneren Schafts positioniert ist,
wobei die erste und zweite Einführvorrichtung so angepasst sind, um in einer benachbarten Konfiguration mit einem Gesamtdurchmesser ausgeführt zu werden und
wobei der Gesamtdurchmesser geringer ist als die Summe aus dem ersten distalen Außendurchmeser und dem zweiten distalen Außendurchmesser.

2. Stent-Abgabesystem nach Anspruch 1, wobei die erste Einführvorrichtung ferner einen ersten Stent mit einem proximalen und einem distalen Ende umfasst, welche an dem ersten Stent aufnehmenden Bereich des ersten inneren Schafts befestigt sind.

3. Stent-Abgabesystem nach Anspruch 2, wobei die erste Einführvorrichtung ferner einen ersten Pusher umfasst, welcher an dem ersten inneren Schaft befestigt ist.

4. Stent-Abgabesystem nach Anspruch 3, wobei das proximale Ende des ersten Stents an den ersten Pusher angrenzt.

5. Stent-Abgabesystem nach einem der vorhergehenden Ansprüche, wobei die erste Einführvorrichtung ferner ein erstes Drahtführungslumen umfasst.

6. Stent-Abgabesystem nach Anspruch 5, wobei das erste Drahtführungslumen sich durch mindestens einen Abschnitt der ersten Einführvorrichtung erstreckt.

7. Stent-Abgabesystem nach Anspruch 5, wobei das erste Drahtführungslumen sich durch mindestens einen Abschnitt des ersten inneren Schafts erstreckt.

8. Stent-Abgabesystem nach Anspruch 5, wobei das erste Drahtführungslumen sich proximal von dem distalen Ende der ersten Einführvorrichtung über eine Strecke von bis zu etwa 20 cm oder bis zu etwa 1 cm erstreckt.

9. Stent-Abgabesystem nach einem der vorhergehenden Ansprüche, wobei die erste Einführvorrichtung ferner eine erste Stentspitze umfasst, welche an dem distalen Ende des ersten inneren Schafts befestigt ist, wobei die erste Stahlspitze vorzugsweise verjüngt ist.

10. Stent-Abgabesystem nach einem der vorhergehenden Ansprüche, wobei der erste proximale äußere Katheter und der erste distale äußere Katheter zwei getrennte Katheter umfassen.

11. Stent-Abgabesystem nach einem der vorhergehenden Ansprüche, wobei die Summe aus dem ersten proximalen Außendurchmesser und dem zweiten distalen Außendurchmesser geringer ist als der Gesamtdurchmesser.

12. Stent-Abgabesystem nach einem der vorhergehenden Ansprüche, wobei die Summe aus dem ersten proximalen Außendurchmesser und dem zweiten distalen Außendurchmesser und mindestens ein Durchmesser von einer ersten und einer zweiten Drahtführung geringer ist als der Gesamtdurchmesser.

13. Stent-Abgabesystem nach einem der vorhergehenden Ansprüche, wobei der erste proximale äußere Katheter angrenzend zu dem zweiten distalen äußeren Katheter ausgeführt ist.

14. Stent-Abgabesystem nach einem der vorhergehenden Ansprüche, wobei der erste proximale Außendurchmesser angrenzend an den zweiten distalen Außendurchmesser ausgeführt ist.

15. Stent-Abgabesystem nach einem der vorhergehenden Ansprüche, wobei der Gesamtdurchmesser etwa 3,0 bis etwa 4,5 mm beträgt.

16. Stent-Abgabesystem nach einem der vorhergehenden Ansprüche, wobei der erste proximale Außendurchmesser etwa 5,0 bis etwa 6,0 French beträgt.

17. Stent-Abgabesystem nach einem der vorhergehenden Ansprüche, wobei der erste distale Außendurchmesser etwa 6,0 bis etwa 7,0 French beträgt.

18. Stent-Abgabesystem nach einem der vorhergehenden Ansprüche, welches ferner umfasst: einen zweiten Stent aufnehmenden Bereich, der an einem distalen Abschnitt des zweiten inneren Schafts positioniert ist, einen zweiten Pusher, der angrenzend an den zweiten Stent aufnehmenden Bereich positioniert ist,
mit einer zweiten Stentspitze, die vorzugsweise an dem distalen Ende des zweiten inneren Schafts befestigt ist, wobei sich dort
ein zweiter Stent mit einem proximalen und einem distalen Ende befindet, welcher vorzugsweise in dem zweiten Stent aufnehmenden Bereich des zweiten inneren Schafts derart befestigt ist, dass das proximale Ende des zweiten Stents an den zweiten Pusher anstößt.

19. Stent-Abgabesystem nach einem der vorhergehenden Ansprüche, welches ferner ein zweites Drahtführungslumen umfasst, welches sich proximal von dem distalen Ende der zweiten Einführvorrichtung durch mindestens einen Abschnitt der zweiten Einführvorrichtung erstreckt.

## Revendications

1. Système d'administration de stent, comprenant :
a) un premier introducteur ayant des extrémités proximale et distale, comprenant :
i) un premier cathéter extérieur ayant des extrémités proximale et distale, le premier cathéter extérieur comprenant :
A) un premier cathéter extérieur proximal ayant des extrémités proximale et distale et un premier diamètre extérieur proximal, et
B) un premier cathéter extérieur distal ayant des extrémités proximale et distale et un premier diamètre extérieur distal,
dans lequel l'extrémité distale du premier cathéter extérieur proximal est connectée à l'extrémité proximale du premier cathéter extérieur distal;
dans lequel le premier diamètre extérieur proximal est inférieur au premier diamètre extérieur distal;
ii) un premier conduit intérieur situé coaxialement dans ledit premier cathéter extérieur, le premier conduit intérieur ayant des extrémités proximale et distale, une première zone de retenue de stent étant située sur une partie distale du premier conduit intérieur; et
b) un deuxième introducteur ayant des extrémités proximale et distale, comprenant :
i) un deuxième cathéter extérieur ayant des extrémités proximale et distale, le deuxième cathéter extérieur comprenant:
A) un deuxième cathéter proximal ayant des extrémités proximale et distale et un deuxième diamètre extérieur proximal; et
B) un deuxième cathéter distal ayant des extrémités proximale et distale et un deuxième diamètre extérieur distal;
dans lequel le deuxième diamètre extérieur proximal est inférieur au deuxième diamètre extérieur distal; et
dans lequel l'extrémité distale du deuxième cathéter proximal est connectée à l'extrémité proximale du deuxième cathéter distal;
ii) un deuxième conduit intérieur situé coaxialement dans ledit deuxième cathéter extérieur, le deuxième conduit ayant des extrémités proximale et distale, une deuxième zone de retenue de stent étant située sur une partie distale du deuxième conduit intérieur;
dans lequel lesdits premier et deuxième introducteurs sont adaptés pour être disposés selon une configuration adjacente ayant un diamètre total; et
dans lequel le diamètre total fait moins que la somme du premier diamètre extérieur distal et du deuxième diamètre extérieur distal.

2. Système d'administration de stent de la revendication 1, dans lequel le premier introducteur comprend en outre un premier stent ayant des extrémités proximale et distale monté sur la première zone de retenue de stent du premier conduit intérieur.

3. Système d'administration de stent de la revendication 2, dans lequel le premier introducteur comprend en outre un premier collier poussoir attaché au premier conduit intérieur.

4. Système d'administration de stent de la revendication 3, dans lequel l'extrémité proximale du premier stent bute contre le premier collier poussoir.

5. Système d'administration de stent de l'une quelconque des revendications précédentes, dans lequel le premier introducteur comprend en outre un premier passage de fil de guidage.

6. Système d'administration de stent de la revendication 5, dans lequel le premier passage de fil de guidage s'étend à travers une partie au moins du premier introducteur.

7. Système d'administration de stent de la revendication 5, dans lequel le premier passage de fil de guidage s'étend à travers une partie au moins du premier conduit intérieur.

8. Système d'administration de stent de la revendication 5, dans lequel le premier passage de fil de guidage s'étend dans le sens proximal à partir de l'extrémité distale du premier introducteur sur une distance de jusqu'à environ 20 cm ou de jusqu'à environ 1 cm.

9. Système d'administration de stent de l'une quelconque des revendications précédentes, dans lequel le premier introducteur comprend en outre une première pointe de stent attachée à l'extrémité distale du premier conduit intérieur, la première pointe de stent étant de préférence conique.

10. Système d'administration de stent de l'une quelconque des revendications précédentes, dans lequel le premier cathéter extérieur proximal et le premier cathéter extérieur distal comprennent deux cathéters séparés.

11. Système d'administration de stent de l'une quelconque des revendications précédentes, dans lequel la somme du premier diamètre extérieur proximal et du deuxième diamètre extérieur distal fait moins que le diamètre total.

12. Système d'administration de stent de l'une quelconque des revendications précédentes, dans lequel la somme du premier diamètre extérieur proximal et du deuxième diamètre extérieur distal et d'au moins l'un d'un premier et d'un deuxième diamètres de fil de guidage, fait moins que le diamètre total.

13. Système d'administration de stent de l'une quelconque des revendications précédentes, dans lequel le premier cathéter extérieur proximal est disposé adjacent au deuxième cathéter extérieur distal.

14. Système d'administration de stent de l'une quelconque des revendications précédentes, dans lequel le premier diamètre extérieur proximal est disposé adjacent au deuxième diamètre extérieur distal.

15. Système d'administration de stent de l'une quelconque des revendications précédentes, dans lequel le diamètre total fait d'environ 3,0 à environ 4,5 mm.

16. Système d'administration de stent de l'une quelconque des revendications précédentes, dans lequel le premier diamètre extérieur proximal fait d'environ 5,0 à environ 6,0 French.

17. Système d'administration de stent de l'une quelconque des revendications précédentes, dans lequel le premier diamètre extérieur distal fait d'environ 6,0 à environ 7,0 French.

18. Système d'administration de stent de l'une quelconque des revendications précédentes, comprenant en outre: une deuxième zone de retenue de stent située sur une partie distale du deuxième conduit intérieur, un deuxième collier poussoir étant situé près de la deuxième zone de retenue de stent;
avec une deuxième pointe de stent attachée de préférence à l'extrémité distale du deuxième conduit intérieur,
un deuxième stent ayant des extrémités proximale et distale étant monté de préférence sur la deuxième zone de retenue de stent du deuxième conduit intérieur de sorte que l'extrémité proximale du deuxième stent bute contre le deuxième collier poussoir.

19. Système d'administration de stent de l'une quelconque des revendications précédentes, comprenant en outre un deuxième passage de fil de guidage s'étendant dans le sens proximal à partir de l'extrémité distale du deuxième introducteur à travers une partie au moins du deuxième introducteur.
